# EUROPEAN PATENT SPECIFICATION

(11) **EP 3 127 024 B1**
(45) Date of publication and mention of the grant of the patent: **19.09.2018**
(21) Application number: 14734218.2
(22) Date of filing: 02.04.2014
(51) Int. Cl.: G16H 50/70

(54) **AUTOMATIC DETERMINATION OF APPROPRIATE MEDICAL PRODUCTS ACCORDING TO WOUND CLASSIFICATION**
AUTOMATISCHE BESTIMMUNG VON ANGEMESSENEN MEDIZINISCHEN PRODUKTEN GEMÄSS WUNDKLASSIFIZIERUNG
DÉTERMINATION AUTOMATIQUE DE PRODUITS MÉDICAUX APPROPRIÉS EN FONCTION D'UNE CLASSE DE BLESSURE

(43) Date of publication of application: 08.02.2017
(73) Proprietor: Laboratoires Urgo, 21300 Chenove (FR)
(72) Inventor: VIVAS, Esther, E-08301 Mataró (Barcelona) (ES); CAMPS, Xavier, E-08023 Barcelona (ES)
(74) Representative: Hirsch & Associés
(86) International application number: PCT/IB2014/000794
(87) International publication number: WO 2015/150852

(56) References cited:
- WO-A1-00/41714
- US-A1- 2009 204 423
- US-A1- 2009 216 553
- US-A1- 2010 241 447

## Description

### FIELD OF THE INVENTION

The invention relates to the medical domain and aims at assisting a person in determining the appropriate products, e.g. dressings, for healing a wound.

### BACKGROUND OF THE INVENTION

Wounds are injuries impacting the tissues of the skin and, sometimes, of the underlying flesh. They can results from various causes: surgical procedure, infectious disease, falls, object or weapon impacts, animal bites, etc.

Consequently, the wounds may be of different sorts and require appropriate healing means adapted to their specificity. A diagnosis is therefore performed by a medical professional in order to characterize the wound and decide on the adapted medical products and healing procedure.

Generally, then, the medical professional decides on the appropriate healing product according to his/her own professional knowledge and expertise. The product consists on an off-the-shelf products package, e.g. dressings' package, which is handed over to the injured person.

Drawback for such a solution is manifold and comprises relying on the expertise of an experienced medical professional and relying on existing off-the-shelf products package.

The major issue about these existing packages is notably that they comprise an important number of products, e.g. dressings, of a same type, whereas a wound can evolve in the time such that a product adapted at the time of the visit may become unappropriated some days later. Health professional are at the same time overwhelmed by the large range of products available in their storehouse and they are not trained for all products. Consequently, most of the products are wasted and the injured person may become soon without any appropriate healing means.

Wound care world is complex and not necessarily well controlled by professional. Costs are also uncontrolled as there is not following of wounds neither of the product consumption per wound.

US 2009/216553 A1 discloses methods involving providing a database of wound dressings and dressing information, observing and recording characteristics of a patient's wound, comparing them to the database, and displaying a list of dressings to a user for treating the wound.

### SUMMARY OF THE INVENTION

The object of the present invention is to alleviate at least partly the above mentioned drawbacks.

More particularly, the invention aims to develop a system which allows billing per wound instead of billing per product. For this purpose a treatment protocol with treatment kits for all types of wounds and treatment phases has been developed. An IT system will be provided as well in order to support nursing staff in the evaluation of the wound, to monitor the wound and to follow wound care costs.

This object is achieved with a method for determining appropriate products adapted for healing a wound, comprising steps of:
- Determining values (p₁, p2 ...p_{N}) for physical properties of said wound, visually or by measurements, and entering said values into an automatic classification system (CS) through a communication terminal (T);
Determining by said automatic classification system a class (C) for said wound corresponding to said values, by forming a vector with said values which identifies a unique class;
Inputting said class into an automatic matching system (MS) for determining an identification (Id) of a kit corresponding to said class according to a database (DB) associating classes for wounds and identifications of kits, each of said kit comprising at least one product adapted for healing said wound;
Providing said identification (Id),
wherein the method further comprises the steps of:
Evaluating by a prediction system at least a future state for said wound and determining subsequent classes corresponding to said at least a future state on the basis of the determined class of the wound;
Inputting said subsequent classes into the automatic matching system (MS);
Determining and providing subsequent identifications (Id₁, ..., Id_{M}) of kits corresponding to said at least a future state.

Preferred embodiments comprise one or more of the following features, which can be taken separately or together, either in partial combination or in full combination.
- said physical parameters correspond to medical characteristics visually determinable by a person;
- said physical parameters are within a group made of: type of wound, signs of infection, bed source, exudation, state of peri-ulceral skin, cavity and size;
- the method further comprises evaluating an evolution for said wound and providing indication for a next visit.
- said identification is provided to a stock management system for delivering said kit.

Another aspect of the invention relates to a computer program product comprising a computer readable medium, having thereon a computer program comprising program instructions, the computer program being loadable into a data processing unit and adapted to cause execution of the method previously described when the computer program is run by the data processing unit.

Still another aspect of the invention concerns a system for assisting a person determining appropriate products adapted for healing a wound, comprising:
- an automatic classification system (CS) adapted to receive values for physical properties of said wound and to determine a classification for said wound corresponding to said values by forming a vector with said values which identifies a unique class
- an automatic matching system (MS) adapted for determining an identification (Id) of a kit corresponding to said class according to a database (DB) associating classes for wounds and identifications of kits, each of said kit comprising at least one product adapted for healing said wound, and for providing said identification (Id) to said person,
wherein the system further comprises a prediction system for evaluating at least a future state for a wound and determining subsequent classifications corresponding to said at least a future state on the basis of a determined classification of the wound.

Preferred embodiments comprise one or more of the following features, which can be taken separately or together, either in partial combination or in full combination.
- said physical parameters correspond to medical characteristics visually determinable by said person.
- said physical parameters are within a group made of: type of wound, infection, bed source, exudation, state of skin, depth and size.
- The system further comprises evaluating an evolution for said wound and providing said person with indication for a next visit.
- said identification is provided to a stock management system for delivering said kit.
- The system further comprises communication means with a terminal adapted to receive values and transmit them to said automatic classification system.

Further features and advantages of the invention will appear from the following description of embodiments of the invention, given as nonlimiting examples, with reference to the accompanying drawings listed hereunder.

### BRIEF DESCRIPTION OF THE DRAWINGS

Fig. 1 shows an example of embodiment for the invention.
Fig. 2 shows an example of physical parameters and possible values.
Fig. 3 shows a tree of possible evolutions for an example wound

### DETAILED DESCRIPTION OF THE INVENTION

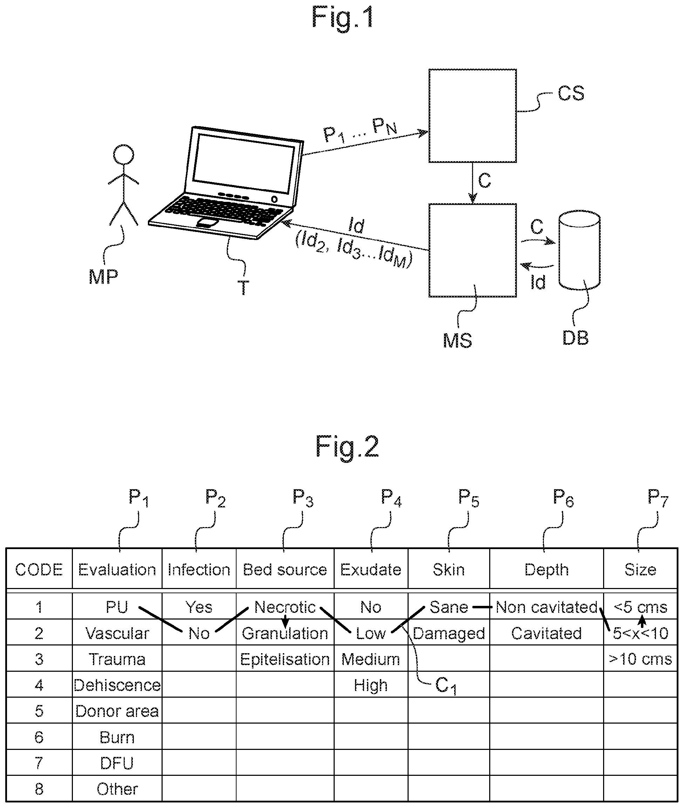

The invention may be deployed within a medical facility and handled by person. FIG. 1 represents an embodiment for a deployment of the invention.

The person MP may be a physician, a nurse, a pharmacist, a physician assistant, or any appropriate person according to the local professional regulations and of the urgent nature of the medical care to be provided. In some situation, the invention may also be deployed on the field, in particular in urgent situations.

An advantage of the invention is that the person is assisted so that the required level of expertise may be lowered.

According to the invention, the person determines values p₁, p₂, p₃...p_{N} of a set of physical properties of the wound to be healed. These physical properties are chosen to allow a meaningful classification of the wound.

Various possible physical properties may be used for doing such a classification.

In a preferred embodiment, these physical properties correspond to medical characteristics which are visually determinable by the person. These physical properties may within a group made of: etiology of the wound, signs of infection, bed source, level of exudate, peri-ulceral skin, depth and size, as depicted on FIG. 2

On FIG. 2, the physical properties p₁, p₂...p_{N} are represented as columns of a table. The rows of the table represent the different values that each property can take.

The first physical parameter p₁ corresponds to the etiology of the wound, which can be, for examples, Pressure Ulcer (PU), vascular, Trauma, Dehiscence, Donor area, Burn, DFU (Diabetic Foot Ulcer), Other. Of course, other typologies of the wound can be designed.

The second physical parameter p₂ corresponds to the visible signs of infection of the wound and it can take only two values: yes or no.

The third physical parameter p₃ correspond to the Bed source and can take as possible values: Necrotic tissue, Sloughy wounds, Granulation or Epitelisation.

The fourth physical parameter p₄ correspond to the level of Exudation, which can take as possible values: No, Low, Medium, High.

The fifth physical parameter p₅ correspond to the aspect of the peri-ulceral skin and can take as values: Healthy or Damaged.

The six physical parameter p₆ correspond to the Cavity of the wound and can take as values: No cavity or Cavity.

The seventh physical parameter p₄ correspond to the size of the wound and can take as values : <10cm² or >10cm²

The possible values that can be taken by each of the physical parameters are examples only. It should be understood that many other possibilities can be designed. For instance, for the size of the wound, p₇, the threshold 5 and 10 cms can be adapted, as well as the number of possible intervals (here 2).

Similarly, in these example, 7 physical parameters (N=7) are used, but the invention may be embodied with fewer or more physical parameters.

The determination of these values can be made clinically by the person by simple observation of the wound.

The observation can be direct, during a visit of the injured person to the medical facility for instance. The observation can also be indirect, for instance on a photography or through a video or other technical means allowing the person to provide medical care on a remote location.

Also, the values of all or some of the physical parameters may be measured thanks to measurement means, in order to acquire better assessment of the wound's characteristic. The type of measurement means depends of course on the physical parameter to measure.

The observed or measured values of the physical parameters can be then entered into the automatic classification system CS, through a communication terminal T.

The communication terminal is any electronic device having a man-machine interface able to input data, and communication means allowing transferring the inputted data to the automatic classification system.

In some embodiments, the automatic classification system CS, the terminal T and the automatic matching system MS are embedded into a same data processing system. The communication means between the terminal and the classification and matching systems are internal communication means, basically software means.

In other embodiments, the automatic matching system MS and the automatic classification system CS are embedded in a first data processing system (a "server"), whereas the terminal T constitutes a distinct, and possibly remote, second processing system. In this case, the communication means maybe any type of networking solution, including Ethernet, Wifi, WLAN, etc.

The terminal T may be a desktop computer, a laptop computer, a smartphone, a tablet, or any other similar devices.

The Man machine interface can be a regular keyboard, a virtual keyboard displayed on a touchscreen, a vocal commanded input mechanism, etc.

The automatic classification system CS receives the values inputted by the person MP, and can then determine a class C for the wound corresponding to these values.

The class can be simply determined by a combination of the values of the physical parameters: the values form a vector which identifies a unique class C.

In the example depicted on FIG. 2, each possible value is assigned to a digital code, shown on the first column "1". In this example, the code ranges from 1 to 8.

Accordingly, each combination of values of the physical parameters p₁, p₂...p₇ can correspond to a vector of such codes. For instance, the combination C1, depicted as a line linking values over each physical parameter, can be written as a vector "1212112".

Such an embodiment simplifies the representation of the data within the automatic classification system and automatic matching system, but obviously other embodiments and data representations are designable by the man skilled in the art.

A class can be assigned to the combination of values in different ways.

For example, the assignment can be straightforward, so that the class C is directly indicated by the vector (e.g. 1212112). Alternatively, a correspondence can be provided, as a mathematical formula (e.g. a hashing mechanism) or matching table.

In the example depicted on FIG. 2, there are 2016 possible vectors, and thus classes.

This class C can then be inputted into the automatic matching system MS for determining an identification Id of a kit corresponding to this class. This determination is based on a database DB associating classes C and identifications of kits.

This database can be provisioned in advance according to the available kits on the market, and may be customized according to the know-how of a responsible person.

For, ideally, all or a substantive part of the possible classes, at least a kit is associated within the database DB. Additional information can also be associated for internal purpose but also for being displayed to the person. This additional information may comprise indication about the severity of the wound, indication about a next visit of the injured person, a usual name for the wound, etc.

Accordingly, the person can be notified in case of wrong evolution of the wound or treatment. If in a determine time the wound is not progressing, the he or she will receive an alert and it will be offered him/her to send the wound data to an expert for advice. Or if the person is using a treatment for a too long or an inappropriate time, he/she will receive an alert for the wrong use, for example the silver treatment should not be applied more than 2 weeks.

The kits comprise at least one product adapted for healing the wound corresponding to the class C.

The product is basically a dressing or a set of dressings, but other products are possible like bandages, creams, balms, etc.

Accordingly, the dressing is accurately adapted to the wound to match its physical parameters (size, etc.)

Examples for kits may be as in the following table:

| Id | Description |
|---|---|
| 1 | Necrotic/sloughy wound - damaged perilesional skin - deep (cavitated) |
| 2 | Necrotic/sloughy wound - deep (cavitated) |
| 3 | Necrotic/sloughy wound |
| 4 | Necrotic/sloughy wound with compression therapy |
| 5 | Granulating wound - medium/high exudate |
| 6 | Granulating wound - medium/high exudate with compression therapy |
| 7 | Granulating wound - medium/high exudate-damaged perilesional skin-Cavitated wound |
| 8 | Granulating wound - Cavitated wound - medium/high exudate |
| 9 | Granulating wound Cavitated wound - low exudate |
| 10 | Granulating wound - Cavitated wound - low exudate with compression therapy |
| 11 | Burns without signs of infection |
| 12 | Burns - Burns without infections signs-medium exudate |
| 13 | Necrotic/sloughy wound - damaged perilesional skin - cavitated wound - high exudate with signs of infection |
| 14 | Necrotic/sloughy wound - cavitated wound - high exudate with signs of infection |
| 15 | Granulating wound - medium/high exudate with signs of infection |
| 16 | Granulating wound - damaged perilesional skin - Cavitated wound - medium/high exudate with signs of infection |
| 17 | Granulating wound - Cavitated wound - medium/high exudate with signs of infection |
| 18 | Burns with signs/risks of infection |

The databases DB makes the association between the classes C and the identification Id of kits.

It can look like in the following example:

| C | Id | Description |
|---|---|---|
| 1211211 | 1 | Necrotic/sloughy wound - damaged perilesional skin - deep (cavitated) |
| 1211111 | 2 | Necrotic/sloughy wound - deep (cavitated) |
| 1212112 | 3 | Necrotic/sloughy wound |
| 1111211 | 4 | Necrotic/sloughy wound with compression therapy |
| 1111111 | 5 | Granulating wound - medium/high exudate |

The identification Id can then be provided to person. For instance, it can be provided through the same terminal T, and by making use of its man-machine interface.

Reading it on the screen of the terminal T, for instance, the person can simply pick up off shelf the kit which is so identified and either use it actually, or hand it over to the injured person.

According to the invention, a prediction system is provided in order to evaluate clinically at least a future state for the wound. Taking as input the class C provided by the automatic classification system CS, it can determine a most probable state for the wound, in the form, for instance, as a subsequent class, or subsequent classes. This prediction is based on professional know-how and can be implemented as an expert system or as a database matching a given class with a subsequent class.

The subsequent class is provided a "most probable class" only, given the fact that the evolution may depart from these prediction under the influence of many factors, including external factors.

These subsequent classes may be inputted into the automatic matching system MS, which can then determine subsequent identifications Id₁, Id₂... Id_{M} of kits corresponding to the future state or states.

In the example depicted on FIG. 2, the combination C1 would probably evolve according to the arrows toward a new combination. This new combination corresponds to the vector "1222111". Turning back to the table provided here-above, this vector "1222111" corresponds to a class C associated within the database DB to the identification "2".

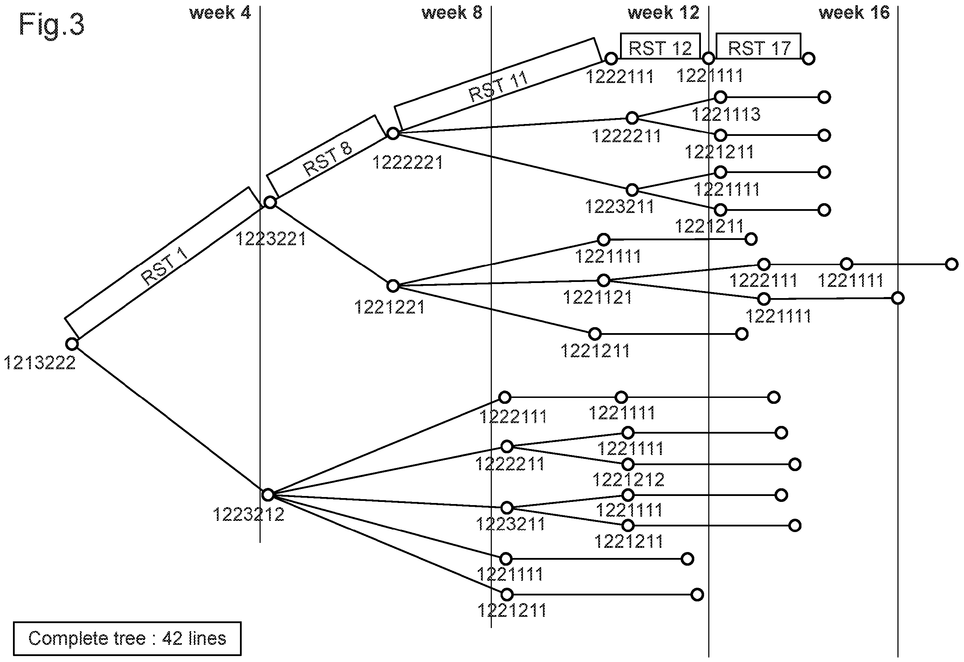

FIG. 3 depicts a tree representing possible evolutions of a wound, for the example of a wound "121322". Each "column" represents an additional depth inside the tree, corresponding to the time (here counted as weeks). Each branch corresponds to a possible evolution leading to a possible combination, associated with a vector

The subsequent identification(s) can then be provided to the person, as previously explained.

The person can then not only pick up the appropriate kit for the present time, but also for the probable future states of the wound. S/he can hand it over to the injured person which can then get dressings appropriate for an extended period of time.

In the example, then, the person can provide to the injured person the kits number 2 and 3.

Consequently, the person is assisted in determining which medical products, e.g. dressings, should be provided by determining simple and visible physical parameters.

In addition, he or she is provided with medical products, e.g. dressings, according to predictions of the evolution of the wound. Therefore, only the appropriate number of products can be provided, and waste of unused medical product is prevented.

For instance, instead of selling packs of dressing, only the appropriate number of dressing can be sold or prescribed, together with indication to use them properly (i.e. which one corresponds to which state of the wound, and which timing).

Another embodiment of the invention consists in establishing a like between the automatic matching system and a stock management system. The identification Id₁, Id₂... Id_{M} are provided to this stock management system which can check availability of the identified kits and trigger delivery to a provided address. Such a stock management system may be linked to an e-commerce solution for billing and payment.

The invention has been described with reference to preferred embodiments. However, many variations are possible within the scope of the invention.

## Claims

1. Method for determining appropriate products adapted for healing a wound, comprising steps of:
Determining values (p₁, p₂...p_{N}) for physical properties of said wound, visually or by measurements, and entering said values into an automatic classification system (CS) through a communication terminal (T);
Determining by said automatic classification system a class (C) for said wound corresponding to said values, by forming a vector with said values which identifies a unique class;
Inputting said class into an automatic matching system (MS) for determining an identification (Id) of a kit corresponding to said class according to a database (DB) associating classes for wounds and identifications of kits, each of said kit comprising at least one product adapted for healing said wound;
Providing said identification (Id),
wherein the method further comprises the steps of:
Evaluating by a prediction system at least a future state for said wound and determining subsequent classes corresponding to said at least a future state on the basis of the determined class of the wound;
Inputting said subsequent classes into the automatic matching system (MS);
Determining and providing subsequent identifications (Id₁, ..., Id_{M}) of kits corresponding to said at least a future state.

2. Method according to the previous claim; wherein said physical parameters are within a group made of: type of wound, signs of infection, bed source, exudation, state of peri-ulceral skin, cavity and size.

3. Method according to any of the previous claims, wherein said identification (Id) and subsequent identifications (Id₁, ..., Id_{M}) are provided to a stock management system for delivering said kits.

4. Computer program product comprising a computer readable medium, having thereon a computer program comprising program instructions, the computer program being loadable into a data processing unit and adapted to cause execution of the method according to any of the claims 1 to 3 when the computer program is run by the data processing unit.

5. System for assisting a person determining appropriate products adapted for healing a wound, comprising:
- an automatic classification system (CS) adapted to receive values for physical properties of said wound and to determine a classification for said wound corresponding to said values by forming a vector with said values which identifies a unique class
- an automatic matching system (MS) adapted for determining an identification (Id) of a kit corresponding to said class according to a database (DB) associating classes for wounds and identifications of kits, each of said kit comprising at least one product adapted for healing said wound, and for providing said identification (Id) to said person,
wherein the system further comprises a prediction system for evaluating at least a future state for a wound and determining subsequent classifications corresponding to said at least a future state on the basis of a determined classification of the wound.

6. System according to claim 5, wherein said physical parameters correspond to medical characteristics visually determinable by said person.

7. System according to the previous claim; wherein said physical parameters are within a group made of: type of wound, infection, bed source, exudation, state of skin, depth and size.

8. System according to any of the claims 5 to 7, wherein said identification (Id) is provided to a stock management system for delivering said kit.

9. System according to any of the claims 5 to 8, further comprising communication means with a terminal (T) adapted to receive values and transmit them to said automatic classification system (CS).

## Patentansprüche

1. Verfahren zum Bestimmen geeigneter Produkte, die zum Heilen einer Wunde ausgelegt sind, die folgenden Schritte umfassend:
Bestimmen von Werten (p₁, p₂...p_{N}) für physikalische Eigenschaften der Wunde, optisch oder durch Messungen, und Eingeben der Werte in ein automatisches Klassifizierungssystem (CS) durch ein Kommunikationsterminal (T);
durch das automatische Klassifizierungssystem, Bestimmen einer Klasse (C) für die Wunde, die den Werten entspricht, durch Bilden eines Vektors mit den Werten, der eine eindeutige Klasse identifiziert;
Eingeben der Klasse in ein automatisches Anpassungssystem (MS) zum Bestimmen einer Kennzeichnung eines Kits, das der Klasse gemäß einer Datenbank (DB) entspricht, die Klassen für Wunden und Identifizierungen von Kits ist verbindet, wobei jedes der Kits mindestens ein Produkt enthält, das zum Heilen der Wunde geeignet ist;
Bereitstellen der Kennzeichnung (Id), wobei das Verfahren ferner die folgenden Schritte umfasst:
durch ein Vorhersagesystem, Bewerten von mindestens einem zukünftigen Zustand für die Wunde und Bestimmen von nachfolgenden Klassen, die mindestens einem zukünftigen Zustand entsprechen, auf der Basis der festgestellten Klasse der Wunde;
Eingeben von nachfolgenden Klassen in das automatische Anpassungssystem (MS);
Bestimmen und Bereitstellen von nachfolgenden Kennzeichnungen (Id₁, ..., Id_{M}) von Kits, die mindestens einem zukünftigen Zustand entsprechen.

2. Verfahren nach dem vorherigen Anspruch; wobei die physischen Parameter innerhalb einer Gruppe liegen, die besteht aus: Art der Wunde, Infektionszeichen, Bettquelle, Exsudation, Zustand der Haut um das Geschwür, Höhlung und Größe.

3. Verfahren nach einem der vorherigen Ansprüche, wobei die Identifikation (Id) und nachfolgende Identifikationen (Id₁, ..., Id_{M}) einem Lagerverwaltungssystem zur Auslieferung der Kits zugeführt werden.

4. Computerprogrammprodukt, welches ein computerlesbares Medium enthält, auf dem sich ein Computerprogramm befindet, das Programmanweisungen umfasst, wobei das Computerprogramm in eine datenverarbeitende Einheit geladen werden kann und dafür ausgelegt ist, das Ausführen des Verfahrens nach einem der Ansprüche 1 bis 3 zu bewirken, wenn das Computerprogramm von der datenverarbeitenden Einheit ausgeführt wird.

5. System zur Unterstützung einer Person, die geeignete Produkte bestimmt, welche zum Heilen einer Wunde geeignet sind, umfassend:
- ein automatisches Klassifizierungssystem (CS), das dafür ausgelegt ist, Werte für physische Eigenschaften der Wunde zu empfangen und eine Klassifikation für die Wunde entsprechend den Werten durch Bilden eines Vektors mit den Werten zu bestimmen, der eine eindeutige Klasse identifiziert,
- ein automatisches Anpassungssystem (MS), das dafür ausgelegt ist, eine Identifikation (Id) eines Kits zu bestimmen, der der Klasse gemäß einer Datenbank (DB) entspricht, die Klassen für Wunden und Identifikationen für Kits verbindet, wobei jedes Kit mindestens ein Produkt enthält, das zum Heilen der Wunde geeignet ist, und die Identifikation (Id) für die Person bereitzustellen,
wobei das System ferner ein Vorhersagesystem zum Bewerten von mindestens einem zukünftigen Zustand für eine Wunde und zum Bestimmen von nachfolgenden Klassifikationen umfasst, die dem mindestens einen zukünftigen Zustand auf der Basis einer festgestellten Klassifikation der Wunde entsprechen.

6. System nach Anspruch 5, wobei die physischen Parameter medizinischen Charakteristika entsprechen, die visuell durch die Person festgestellt werden können.

7. System nach dem vorherigen Anspruch; wobei die physischen Parameter innerhalb einer Gruppe liegen, die aus Folgendem besteht: Arzt der Wunde, Infektion, Bettquelle, Exsudation, Zustand der Haut, Tiefe und Größe.

8. System nach einem der Ansprüche 5 bis 7, wobei die Identifikation (Id) einem Lagerverwaltungssystem zur Auslieferung des Kits zugeführt wird.

9. System nach einem der Ansprüche 5 bis 8, das ferner Kommunikationsmittel mit einem Terminal (T) umfasst, die dafür ausgelegt sind, Werte zu empfangen und sie zum automatischen Klassifizierungssystem (CS) zu übertragen.

## Revendications

1. Procédé pour déterminer des produits appropriés conçus pour cicatriser une blessure, comprenant les étapes :
- de détermination de valeurs (p₁, p₂ ... p_{N}) pour les propriétés physiques de ladite blessure, visuellement ou par des mesures, et d'entrée desdites valeurs dans un système de classification automatique (CS) par l'intermédiaire d'un terminal de communication (T) ;
- de détermination, par ledit système de classification automatique, d'une catégorie (C) pour ladite blessure correspondant auxdites valeurs, en formant un vecteur avec lesdites valeurs qui identifie une catégorie unique ;
- d'entrée de ladite catégorie dans un système de mise en correspondance automatique (MS) pour déterminer une identification (Id) d'un kit correspondant à ladite catégorie conformément à une base de données (DB) associant les catégories pour les blessures et les identifications des kits, chacun desdits kits comprenant au moins un produit conçu pour cicatriser ladite blessure ;
- de fourniture de ladite identification (Id), dans lequel le procédé comprend en outre les étapes :
o d'évaluation, par un système de prédiction, d'au moins un état futur pour ladite blessure et de détermination de catégories suivantes correspondant audit au moins un état futur sur la base de la catégorie déterminée de la blessure ;
o d'entrée desdites catégories suivantes dans le système de mise en correspondance automatique (MS);
o de détermination et de fourniture des identifications suivantes (Id₁,..., Id_{M}) des kits correspondant audit au moins un état futur.

2. Procédé selon la revendication précédente, dans lequel lesdits paramètres physiques sont dans un groupe constitué : d'un type de blessure, de signes d'infection, d'une source de lit, d'une exsudation, d'un état de peau péri-ulcérale, d'une cavité et d'une taille.

3. Procédé selon l'une quelconque des revendications précédentes, dans lequel ladite identification (Id) et lesdites identifications subséquentes (Id₁, ..., Id_{M}) sont fournies à un système de gestion de stock pour délivrer lesdits kits.

4. Produit-programme d'ordinateur comprenant un support pouvant être lu par un ordinateur, comportant sur celui-ci un programme d'ordinateur comprenant des instructions de programme, le programme d'ordinateur pouvant être chargé dans une unité de traitement de données et étant conçu pour provoquer l'exécution du procédé selon l'une quelconque des revendications 1 à 3 lorsque le programme d'ordinateur est exécuté par l'unité de traitement de données.

5. Système pour aider une personne à déterminer des produits appropriés conçus pour cicatriser une blessure, comprenant :
- un système de classification automatique (CS) conçu pour recevoir des valeurs pour les propriétés physiques de ladite blessure et pour déterminer une classification pour ladite blessure correspondant auxdites valeurs en formant un vecteur avec lesdites valeurs qui identifie une catégorie unique ;
- un système de mise en correspondance automatique (MS) conçu pour déterminer une identification (Id) d'un kit correspondant à ladite catégorie conformément à une base de données (DB) associant les catégories pour les blessures et les identifications des kits, chacun desdits kits comprenant au moins un produit conçu pour cicatriser ladite blessure, et pour fournir ladite identification (Id) à ladite personne,
dans lequel le système comprend en outre un système de prédiction pour évaluer au moins un état futur pour une blessure et déterminer des classifications suivantes correspondant audit au moins un état futur sur la base d'une classification déterminée de la blessure.

6. Système selon la revendication 5, dans lequel lesdits paramètres physiques correspondent à des caractéristiques médicales pouvant être déterminées visuellement par ladite personne.

7. Système selon la revendication précédente, dans lequel lesdits paramètres physiques sont dans un groupe constitué : d'un type de blessure, d'une infection, d'une source de lit, d'une exsudation, d'un état de peau, d'une profondeur et d'une taille.

8. Système selon l'une quelconque des revendications 5 à 7, dans lequel ladite identification (Id) est fournie à un système de gestion de stock pour délivrer ledit kit.

9. Système selon l'une quelconque des revendications 5 à 8, comprenant en outre des moyens de communication avec un terminal (T) conçu pour recevoir des valeurs et les transmettre audit système de classification automatique (CS).
